# EUROPÄISCHE PATENTSCHRIFT

(11) **EP 1 488 752 B1**
(45) Veröffentlichungstag und Bekanntmachung des Hinweises auf die Patenterteilung: **03.07.2013**
(21) Anmeldenummer: 04010738.5
(22) Anmeldetag: 06.05.2004
(51) Int. Cl.: A61B 17/72, A61B 17/78

(54) **Knochennagel, insbesondere proximaler Femurnagel**
Intramedullary nail, in particular for the neck of the femur
Clou intramédullaire, en particulier pour le col du fémur

(30) Priorität: 18.06.2003 DE 20309399 U
(43) Veröffentlichungstag der Anmeldung: 22.12.2004
(73) Patentinhaber: Stryker Trauma GmbH, 24232 Schönkirchen (DE)
(72) Erfinder: Zander, Nils, 24340 Eckernförde (DE); Cremer, Axel, 23795 Fahrenkrog (DE)
(74) Vertreter: Maiwald Patentanwalts GmbH

(56) Entgegenhaltungen:
- EP-A- 0 321 170
- EP-A- 1 175 872
- WO-A-03/094763
- GB-A- 2 209 947

## Beschreibung

Die Erfindung bezieht sich auf einen Knochennagel, insbesondere proximalen Femurnagel nach dem Oberbegriff des Patentanspruchs 1.

Es ist bekannt, zur Versorgung trochanterer Frakturen des Femurs einen Verriegelungsnagel von proximal in den Femur einzutreiben und den Nagelschaft mit einer schrägen Querbohrung zu versehen, durch welche hindurch eine Schenkelhalsschraube geführt ist. Es ist auch bekannt, in den Verriegelungsnagel einen Verriegelungsstift einzuschrauben, der mit der Schenkelhalsschraube zusammenwirkt, um diese axial und/oder in Drehrichtung wahlweise zu fixieren.

In Verbindung mit einem derartigen Implantat ist auch bekannt geworden, zwei unter einem Winkel den Nagel durchdringende parallele Schenkelhalsschrauben vorzusehen. Die oberste der beiden Schrauben wird durch einen in der Achse des Nagelanschlusses angebrachten Verriegelungsstift an einem axialen Verrutschen gehindert.

WO 03/094763 A1 betrifft einen Knochennagel mit einem Nagelschaft mit einer Querbohrung und einer axialen Bohrung, einer Schraube, die in die Querbohrung einführbar ist, und einen Verriegelungsstift zum Festlegen der Schraube.

EP 1 175 872 A2 (Basis für den Oberbegriff des Anspruchs 1) betrifft einen Knochennagel mit einem Nagelschaft und zwei Querbohrungen zum Einführen von Schrauben mit glatten Schaftabschnitten.

Der Erfindung liegt die Aufgabe zugrunde, einen Knochennagel, insbesondere der zuletzt genannten Art, dahingehend zu verbessern, dass eine wirksame axiale Festlegung einer den Knochennagel quer durchsetzenden Schraube erhalten wird, ohne diese zu beschädigen.

Diese Aufgabe wird durch die Merkmale des Patentanspruchs 1 gelöst.

Bei dem erfindungsgemäßen Knochennagel ist zumindest der Endabschnitt des Verriegelungsstiftes aus einem elastisch und/oder plastisch verformbaren Material geformt.

Nach einer Ausgestaltung der Erfindung besteht der Verriegelungsstift oder zumindest sein Endabschnitt aus Kunststoffmaterial. Alternativ sieht eine Ausgestaltung der Erfindung vor, daß der Endabschnitt aus einem elastisch verformbaren Material besteht, beispielsweise aus einem geeigneten Metall, wobei der Endabschnitt so geformt ist, daß in jeder Drehlage des Verriegelungsstiftes der mit der Schraube in Eingriff tretende Bereich elastisch nachgebend verformt wird.

Die Ausbildung des elastisch verformbaren Eingriffbereiches kann auf verschiedene Art und Weise vorgenommen werden. Hierzu sieht eine Ausgestaltung der Erfindung vor, daß der Verriegelungsabschnitt einen scheibenförmigen Abschnitt aufweist, der einen axialen Abstand zum benachbarten Teil des Endabschnitts aufweist und mit einem mittigen Kernabschnitt des Verriegelungsstiftes verbunden ist. Der Scheibenrand, der mit dem Schaft der Schenkelhalsschraube oder einer anderen Verriegelungsschraube in Eingriff tritt, ist vorzugsweise gerundet, damit er sich nicht in das Material der Schenkelhalsschraube eingräbt.

Vorzugsweise sind mehrere parallele axial beabstandete Scheibenabschnitte vorgesehen, die mit dem Kernabschnitt verbunden sind. Dabei kann der Kernabschnitt vorzugsweise vom freien Ende des Verriegelungsstiftes zum anderen Ende hin in seinem Durchmesser allmählich zunehmen.

Nach einer anderen Ausgestaltung der Erfindung ist vorgesehen, daß der Verriegelungsstift eine Anschlagfläche am Umfang aufweist, die mit einem Anschlag in der axialen Bohrung des Nagels zusammenwirkt. Hierdurch ist ein Begrenzungsanschlag geschaffen, wobei trotz des festen Anschlages und vorhandener Toleranzen stets eine sichere Verriegelung der Schenkelhalsschraube erfolgen kann.

Nach einer anderen Ausgestaltung der Erfindung weist der Verriegelungsstift an dem dem Endabschnitt entgegengesetzten Ende einen glatten Abschnitt auf, der annähernd passend in der Bohrung des Nagelschaftes einsitzt und annähernd mit dem Ende des Nagelschaftes bündig abschließt, wenn der Verriegelungsstift in Eingriff ist bzw. am Anschlag in der Bohrung anliegt. Der Außengewindeabschnitt des Verriegelungsstiftes liegt zwischen dem glatten Abschnitt und dem Endabschnitt.

Die Erfindung soll nachfolgend anhand eines in Zeichnungen dargestellten Ausführungsbeispiels näher erläutert werden.
- Fig. 1: zeigt einen Verriegelungsnagel nach der Erfindung,
- Fig. 2: zeigt die Seitenansicht eines Verriegelungsstiftes für den Verriegelungsnagel nach Fig. 1,
- Fig. 3: zeigt einen Schnitt durch den Verriegelungsstift nach Fig. 2 entlang der Linie 3-3,
- Fig. 4: zeigt vergrößert den Teil 4 nach Fig. 3.

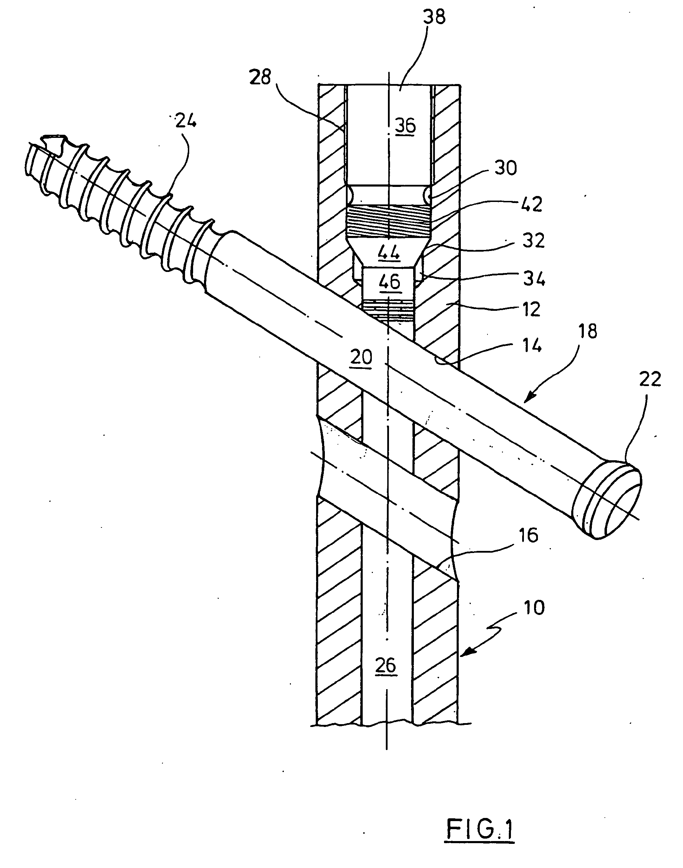

In Fig. 1 ist das proximale Ende eines Femur-Verriegelungsnagels 10 im Schnitt gezeigt. Der nicht gezeigte distale Teil weist mindestens eine Querbohrung auf für die Aufnahme einer Verriegelungsschraube, wie an sich bekannt. Der proximale Abschnitt 12 des Nagelschaftes weist zwei parallele Schrägbohrungen 14, 16 auf, die jeweils eine Schenkelhalsschraube aufnehmen, von denen eine bei 18 gezeigt ist. Die Schenkelhalsschraube 18 weist einen relativ langen, glatten Schaftabschnitt 20 auf, an dessen einem Ende ein Kopf 22 angeordnet ist und an dessen anderem Ende sich ein Gewindeabschnitt 24 anschließt. Das Gewinde 24 ist selbstschneidend oder selbstfurchend. An dem Kopf 22 sind entsprechende Mittel vorgesehen für den Eingriff eines Drehwerkzeugs (nicht gezeigt).

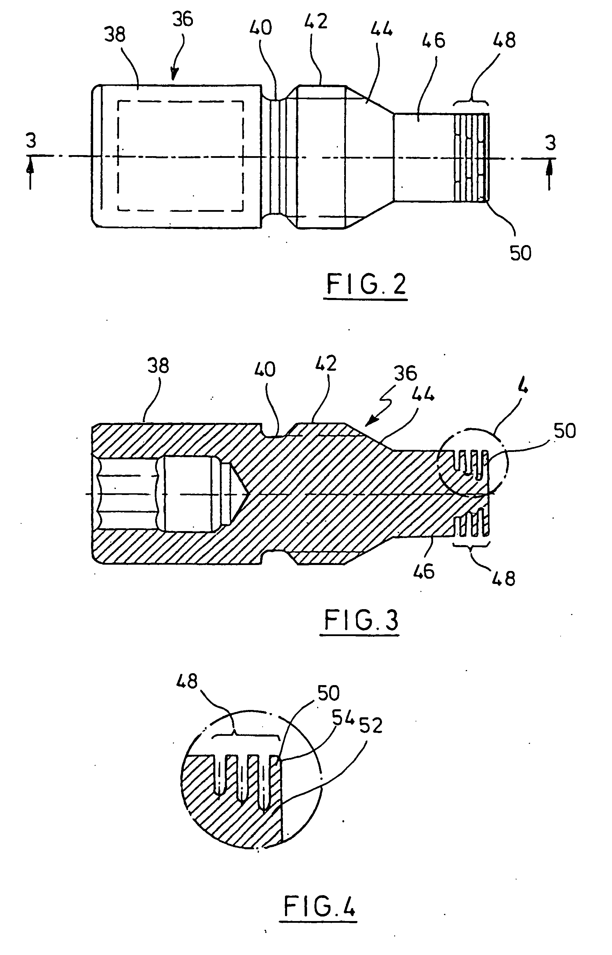

Der Nagelschaft 10 weist eine axiale Bohrung 26 auf, die zum proximalen Ende hin aufgeweitet ist. Dort weist sie einen glatten Bohrungsabschnitt 28, einen Gewindeabschnitt 30, eine konische Anschlagfläche 32 sowie einen im Durchmesser verringerten Abschnitt 34 auf. Die zuletzt beschriebenen Bohrungsabschnitte nehmen einen Verriegelungsstift 36 auf, der anhand der Fign. 2 bis 4 näher beschrieben werden soll.

Der Verriegelungsstift 36 weist einen glatt zylindrischen Abschnitt 38 auf, an den sich nach rechts eine Ringnut 40 anschließt und an diese ein Außengewindeabschnitt 42. An den Außengewindeabschnitt 42 schließt sich ein konischer Abschnitt 44 an, der in einen zylindrischen Abschnitt 46 übergeht. Am zylindrischen Abschnitt 46 sind lamellenartig drei Scheiben 48 angeformt, wie dies deutlicher aus Fig. 4 hervorgeht.

Die einzelnen Scheiben 50 der Scheibenanordnung 48 sind kreisförmig und in einem vorgegebenen gleichmäßigen Abstand voneinander entfernt. Sie sind mit einem mittigen Kernabschnitt 52 verbunden, der zum glatten Abschnitt 38 hin in seinem Durchmesser allmählich zunimmt. Der endseitige Scheibenabschnitt weist eine gerundete Endkante auf, wie bei 54 zu erkennen.

Beim Einsetzen des Verriegelungsstiftes 36 in den Nagelschaftabschnitt 12 wirkt der Gewindeabschnitt 42 des Verriegelungsstiftes 36 mit dem Innengewindeabschnitt 30 des Nagelschaftes 12 zusammen. Der Stift 36 wird soweit eingeschraubt, bis sich die Anschlagfläche des konischen Abschnitts 44 gegen die Anschlagfläche 32 des Nagelschaftes 12 stützt. Kurz vorher kommen die vorderen Scheibenabschnitte 50 mit dem glatten Schaftabschnitt 20 der Schenkelhalsschraube 18 in Eingriff und werden dabei verformt, wie dies deutlich in Fig. 1 zu erkennen ist. Durch diese Verformung wird ermöglicht, daß trotz Toleranzen ein Verriegelungseingriff erzielt wird, wenn die Anschlagflächen von Verriegelungsstift 36 und Nagel 10, 12 in Eingriff sind. Der Verriegelungseingriff ist derart, daß ein relativ großer Flächenteil der Scheibenabschnitte 50 am Umfang des Schaftabschnitts 20 der Schenkelhalsschraube 18 anliegt und dadurch für einen wirksamen Kraftschluß sorgt. Dieser Eingriff ist derart, daß der Schaftabschnitt 20 nicht beschädigt wird.

Der Verriegelungsstift 36 ist aus einem geeigneten elastischen, jedoch körperverträglichen Material.

Es ist einleuchtend, daß der Verriegelungsstift 36 auch zweiteilig geformt sein kann mit einem metallischen Teil für den glatten Abschnitt 36 und den Gewindeabschnitt 42, und einem Teil aus Kunststoff für den Abschnitt 46 und die Scheibenabschnitte 50. Der gesamte Verriegelungsstift 36 kann gegebenenfalls aus Kunststoffmaterial geformt sein.

## Patentansprüche

1. Knochennagel, insbesondere proximaler Femurnagel, mit
einem Nagelschaft, der nahe dem einen Ende mindestens eine Querbohrung (14) aufweist und eine axiale Bohrung (26) mit einem Gewindeabschnitt (30),
einer Schraube (18) mit einem Gewindeabschnitt (24) und einem glatten Schaftabschnitt (20) zwischen dem Gewindeabschnitt und einem Schraubenkopf (22), die in die Querbohrung einführbar ist, und
einem Verriegelungsstift (36) mit einem Außengewindeabschnitt (43), der mit dem Innengewindeabschnitt der Bohrung zusammenwirkt, und einem Endabschnitt (46), **dadurch gekennzeichnet, dass** der Endabschnitt (46) aus elastischem und/oder plastisch verformbarem Material geformt ist und einen scheibenförmigen Abschnitt (50) aufweist, der einen axialen Abstand zum benachbarten Teil des Endabschnitts (46) aufweist und mit einem mittigen Kernabschnitt (52) verbunden ist, wobei der scheibenförmige Abschnitt (50) des Endabschnitts (46) in jeder Drehlage des Verriegelungsstiftes am glatten Schaft der Schraube angreift, um diese axial festzulegen.

2. Knochennagel nach Anspruch 1, wobei für den Verriegelungsstift (36) oder den Endabschnitt (46) Kunststoffmaterial vorgesehen ist.

3. Knochennagel nach Anspruch 1, wobei der Verriegelungsstift (36) aus elastischem Material geformt ist.

4. Knochennagel nach Anspruch 1, wobei mehrere parallele axial beabstandete Scheibenabschnitte (50) vorgesehen sind, die mit dem Kernabschnitt (52) verbunden sind.

5. Knochennagel nach Anspruch 4, wobei die Scheibenabschnitte (50) annähernd den gleichen Außendurchmesser aufweisen und der Kernabschnitt (52) vom freien Ende des Endabschnitts fort allmählich größer wird.

6. Knochennagel nach einem der Ansprüche 1 bis 5, wobei der Verriegelungsstift (36) eine Anschlagfläche (44) aufweist, die an einem Anschlag (32) in der axialen Bohrung (26) angreift, wenn der Verriegelungsstift (36) in der Verriegelungsstellung ist.

7. Knochennagel nach Anspruch 6, wobei der Verriegelungsstift (36) an dem dem Endabschnitt entgegengesetzten Ende einen glatten Abschnitt aufweist und der Außengewindeabschnitt (42) zwischen dem Endabschnitt (46) und dem glatten Abschnitt liegt.

8. Knochennagel nach einem der Ansprüche 1 bis 7, wobei die Schraube (20) eine Schenkelhalsschraube ist.

9. Knochennagel nach Anspruch 8, wobei der Nagelschaft (12) zwei axial beabstandete Querbohrungen (14, 16) aufweist für zwei parallele Schenkelhalsschrauben.

## Claims

1. A bone nail, particularly a proximal femoral nail, with a nail shaft that has at least one transverse bore (14) close to one end thereof and an axial bore (26) with a threaded section (30),
a screw (18) with a threaded section (24) and a smooth shaft section (20) between the threaded section and a screw head (22) that can be inserted in the transverse bore, and
a locking pin (36) with an external threaded section (42) that cooperates with the internal threaded section of the bore, and an end section (46), **characterised in that** the end section (46) is formed from elastically and/or plastically deformable material and has a disc-shaped section (50) that is axially separated from the adjacent portion of the end section (46) and connected to a central core section (52), wherein the disc-shaped section (50) of the end section (46) engages with the smooth shaft of the screw to fix it axially regardless of the rotational position of the locking pin.

2. The bone nail according to claim 1, wherein plastic material is provided for the locking pin (36) or end section (46).

3. The bone nail according to claim 1, wherein the locking pin (36) is formed from elastic material.

4. The bone nail according to claim 1, wherein a plurality of parallel disc sections (50) are arranged with axial separation and are connected to the core section (52).

5. The bone nail according to claim 4, wherein the outer diameters of the disc sections (50) are approximately the same and the core section (52) gradually becomes larger starting from the free end of the end section.

6. The bone nail according to any of claims 1 to 5, wherein the locking pin (36) has a stopper surface (44) that engages with a stopper (32) in the axial bore (26) when the locking pin (36) is in the locking position.

7. The bone nail according to claim 6, wherein the locking pin (36) has a smooth section at the opposite end to the end section, and the external thread section (42) is located between the end section (46) and the smooth section.

8. The bone nail according to any of claims 1 to 7, wherein the screw (20) is a femoral neck screw.

9. The bone nail according to claim 8, wherein the nail shaft (12) has two axially separated transverse bores (14, 16) for two parallel femoral neck screws.

## Revendications

1. Broche osseuse, en particulier broche fémorale proximale, comprenant
une tige de broche, qui présente au voisinage de l'une des extrémités au moins un alésage transversal (14) et un alésage axial (26) avec une section filetée (30),
une vis (18) avec une section filetée (24) et une section de tige lisse (20) entre la section filetée et une tête de vis (22), laquelle vis peut être introduite dans l'alésage transversal, et
une tige de verrouillage (36) avec une section de filetage extérieur (42), qui coopère avec la section filetée de l'alésage, et une section d'extrémité (46), **caractérisée en ce que** la section d'extrémité (46) est formée d'un matériau déformable élastiquement et/ou plastiquement et présente une section (50) en forme de disques, qui présente une distance axiale par rapport à la partie voisine de la section d'extrémité (46) et est assemblée avec une section de coeur centrale (52), la section en forme de disques (50) de la section d'extrémité (46) agissant dans chaque position de rotation de la tige de verrouillage sur la tige lisse de la vis, afin de fixer cette dernière dans la direction axiale.

2. Broche osseuse selon la revendication 1, de la matière plastique étant prévue pour la tige de verrouillage (36) ou la section d'extrémité (46).

3. Broche osseuse selon la revendication 1, la tige de verrouillage (36) étant formée d'un matériau élastique.

4. Broche osseuse selon la revendication 1, dans laquelle sont prévues plusieurs sections de disques (50) parallèles, distantes dans la direction axiale, lesquelles sections sont assemblées avec la section de coeur (52).

5. Broche osseuse selon la revendication 4, les sections de disques (50) présentant approximativement le même diamètre extérieur et la section de coeur (52) devenant graduellement plus grande à l'écart de l'extrémité libre de la section d'extrémité.

6. Broche osseuse selon l'une des revendications 1 à 5, la tige de verrouillage (36) présentant une surface de contact (44), qui agit sur une butée (32) dans l'alésage axial (26), lorsque la tige de verrouillage (36) est dans la position de verrouillage.

7. Broche osseuse selon la revendication 6, la tige de verrouillage (36) présentant une section lisse sur l'extrémité opposée à la section d'extrémité et la section de filetage extérieur (42) se situant entre la section d'extrémité (46) et la section lisse.

8. Broche osseuse selon l'une des revendications 1 à 7, la vis (20) étant une vis pour col du fémur.

9. Broche osseuse selon la revendication 8, la tige de broche (12) présentant deux alésages transversaux (14, 16), distants dans la direction axiale, pour deux vis parallèles pour col du fémur.
